Europäisches Patentamt

(19) European Patent Office  (11) Publication number: **0 093 123**

Office européen des brevets  **B1**

(12) **EUROPEAN PATENT SPECIFICATION**

(45) Date of publication of patent specification: **07.01.87**  (51) Int. Cl.⁴: **A 23 K 1/175, A 61 K 33/00**

(21) Application number: **82903178.0**

(22) Date of filing: **29.10.82**

(86) International application number:
**PCT/HU82/00056**

(87) International publication number:
**WO 83/01559 11.05.83 Gazette 83/11**

(54) **PROCESS FOR THE PREPARATION OF A COMPOSITION SUITABLE FOR PROMOTING THE UTILIZATION (DIGESTIBILITY) OF FODDER BY THE ANIMALS.**

(30) Priority: **02.11.81 HU 325081**

(43) Date of publication of application:
**09.11.83 Bulletin 83/45**

(45) Publication of the grant of the patent:
**07.01.87 Bulletin 87/02**

(84) Designated Contracting States:
**DE GB**

(56) References cited:
**WO-A-81/02242
DE-A-2 755 709
DE-C-1 259 689
FR-A-2 049 322
GB-A- 947 421
GB-A-1 080 299
GB-A-1 494 295
GB-A-1 498 832**

The file contains technical information
submitted after the application was filed and
not included in this specification

(73) Proprietor: **VETOMAGTERMELTETOES
ERTEKESITO VALLALAT
Rottenbiller u. 33
H-1077 Budapest (HU)**

(72) Inventor: **BERES, József
Baross u. 3
H-4600 Kisvárda (HU)**
Inventor: **KLENCZNER, Imre
Sóstói u. 50
H-4400 Nyiregyháza (HU)**
Inventor: **PUSKAS, Lajos
Öz-kös 53. II/1
H-4400 Nyiregyháza (HU)**

(74) Representative: **Hansen, Bernd, Dr.rer.nat. et al
Hoffmann, Eitle & Partner Patentanwälte
Arabellastrasse 4
D-8000 München 81 (DE)**

## Description

Technical field

The invention relates to a process for the preparation of a composition suitable for promoting the utilization (digestibility) of fodder by animals, therefore the composition can be used for the prevention of the impairment of health of alimentary origin.

Background art

In Australia, South America, Sweden, Denmark, the Netherlands and the German Federal Republic even some decades ago it has been noticed that on certain pasture lands the animals show symptoms of unknown ailments.

From the examination concerning these ailments it turned out that they are caused by the reduced amounts or the absence of certain bioinorganic elements of the pasture lands (or fodder) and advantageous results can be achieved by supplying the missing elements. The following articles relate to these examinations: J. F. Filmer and E. J. Underwood, Enzootic Marasmus, treatment with Limonite Fractions, Australian Veterinary Journ. Vol. X., Nr. 3. (1934); J. F. Filmer and E. J. Underwood, Vasting disease. Diagnosis, prevention and treatment, Journ. Dept. Agroc. Western Australia, Vol. 13, Nr. 2. (1936); J. F. Filmer and E. J. Underwood, Enzootic Marasmus. Further data concerning the potency of cobalt as a curative and prophylactic agent, Australian Veterinary Journ. April, (1937); W. Häfele, Die Semperkrankheit, eine Ernährungs- und Entwicklungsstörung des Rindes im Hochschwarzwald in der Umgebung von St. Blasien. Diss. der Vetmed., Fakultät der Universität München (1952); Laatsch, W., Die Schwermetallernährung der Weidopflanzen in Schleswig-Holstein, Schriftenreihe der landwirtschaftlichen Fakultät der Universität Kiel, Heft 10. (1954); Scharrer, K., Biochemie der Spurenelemente, Paul Parey in Berlin und Hamburg (1935).

In Hungary similar symptoms were noticed after the second world war. These symptoms appeared in the often appalling reduction in breeding and production capacity of animals kept and fed under wholesale commercial conditions and they were particularly striking in the case of the very productive breeds of animals (Horváth, Z. and B. Nacsev: Feed injuries deficiency diseases, Mezőgazdasági Kiadó, Budapest, 1972).

That the damage is of great importance is seen in the fact that in the years 1962—1963 the cost value of the deaths caused by pig anaemia was assessed in more than 7 million forints. The same disease in Switzerland, caused a loss of 50 million Swiss francs and in the German Federal Republic 40 million German marks were lost annually.

A great loss is caused also by the reduction of the lambing of sheep and of the vitality of lambs and the loss is further increased by the so called "stinking lameness". In the case of poultry, amongst other symptoms, the level of the egg output is very low, the required weight of broiler chickens can only be achieved later or by the administration of more fodder and the hatchability of the eggs is poor. In the case of Holstein-Friz cattles breeding paralysis can be noticed. Generally the specific amount of the fodder required is high and the ratio of utilization of the fodder is disadvantageous.

The problems could not be solved by the use of fodder or premix compositions having a high vitamin content, moreover the situation was worsened by the use of a higher amount of calcium and phosphorus in the course of feeding. All these problems are ascribable to the fact that other conditions necessary for the metabolic functions which are responsible for the utilization of fodder in the organism, e.g. the satisfactory supply with bioinorganic elements are neglected. The maximal production for the collaboration of the functions is needed, wherein the bioinorganic elements—beside their other role—are activators of essential enzymes.

GB—A—947 421 describes the use of e.g. Fe, Co, Cu; Mn, Zn in feedstuffs for ruminants in the form of chelates; DE—A—2755709 discloses the use of Mg and V as metallo-proteinates to increase the content of vital divalent metals in the tissue of animals. None of these references discloses, however, the combination of elements in a specific chelate or complex form to allow complete disolution thereof.

It is to be mentioned that in the case of some of these elements, the best and long lasting results cannot be achieved when a high dose of the elements is used for a short time but the elements should be used simultaneously in the form of a composition suitable for absorption and their complexes should be formed with organic ligands which can be transformed and used in the vital processes of the organism. When the elements are used daily in small amounts and in the advantageous ratio, the "repositories" of the organism are filled but the elements do not accumulate in toxic amounts and do not become toxic, furthermore synergistic effect may occur.

Disclosure of invention

In the course of the elaboration of the composition of the invention the analysis results of fodder seeds and rough fodder, of the eggs of poor or good hatchability and of the serum and liver of animals having normal or reduced production capacity and the absorption rate of some bioinorganic elements have been taken into consideration.

The effects of the composition according to the invention are the following:

In addition to the prevention of sterility of alimentary origin it also terminates sterility because it preserves or restores the normal functions of the cells of the endocrine glands and also the function of the sexual organs when they have not been injured irreversibly.

It enhances the co-ordinated operation of several enzymes. The activation of the enzymes which carry out the energy producing and synthetic processes and the "respiratory chain" in the mitochondria (cytochrom oxidase, succinate dehydrogenase) is of special importance.

It prevents the development of ataxia, osteoporosis, osteomalacia and paralysis, it helps the co-ordination of the muscular motions and it enhances the function of osteoblasts in helping the osteogenesis, it helps the effectivity of vitamin D and the incorporation of phosphorus into the skeleton of the organism. It has antianaemic activity because it enhances the haemoglobin formation, stimulates the erythropoiesis, improves the intake and transport of oxygen and the oxygen supply of the cells. It enhances the resistance of the organism as it makes the immun reactions more effective, that is, it increases the synthesis of γ-globulins and the antibody production and stimulates the pagocytal capacity of the leucocytes.

It hinders the inflammation processes because it shows antihistamine activity as well.

It hinders the pathological accumulation of cholesterol, fatty acids and fat content of the serum.

It exerts positive inotropic effect on the ventricular muscle of the animals which is caused by the activation of adenylate cyclase as the cyclic AMP level is increased. Thereby also the load-bearing capacity of the organism is increased.

Summarizing the above it can be stated that it enhances the maximal utilization of the ingested fodder, thereby the required specific fodder amount is decreased and the production and breeding capacity of the animals which are characteristic to the species are increased.

The inventive process for the preparation of a composition suitable for promoting the utilization of fodder and preventing the impairment of health of alimentary origin of the animals, comprises blending cobalt or vanadium succinate or cobalt or vanadium ethyl succinate, succinimide nickel complex, iron, magnesium or zinc succinate, boric complex of a lower aliphatic polyhydroxy compound, copper or zinc complex of a monoaminomonocarboxylic acid and manganese complex of an aminopolycarboxylic acid, optionally in the presence of a solvent, and processing the mixture into an orally administerable form.

In the above process as a cobalt compound preferably cobalt chloride or cobalt sulphate or a hydrate thereof is used as a vanadium compound, preferably ammonium metavanadate or sodium metavanadate. In order to form an organic complex of cobalt and vanadium succinic anhydride or the ethyl hydrogen ester thereof is used.

As a nickel compound preferably nickel sulphate or the hydrate thereof is used. In order to form an organic complex of nickel succinimide is used.

As a copper compound preferably copper sulphate and as a zinc compound preferably zinc sulphate or the hydrates thereof can be used. For the preparation of a copper and zinc complex an amphoteric monoaminomonocarboxylic acid (amino acetic acid), preferably glycine is used as a ligand.

As a manganese compound preferably manganese sulphate or a hydrate thereof is used. For the preparation of the manganese complex preferably the sodium salt of EDTA can be used as an amino polycarboxylic acid.

As an iron compound preferably ferrous sulphate and as a magnesium compound preferably magnesium sulphate can be used and in this case as a zinc compound preferably zinc sulphate is employed. Also the hydrates of these compounds can be used.

Best mode of carrying out the invention

According to a preferable form of realization of the process according to the invention as a ligand for iron, magnesium and a part of the zinc succinic acid is employed which is one of the metabolites of the living organism. By the appropriate choice of the amount of succinic acid magnesium, zinc and iron not bound co-ordinately remain in the solution.

From said elements boron is used, as boric acid but its hydrate can be employed as well. As a polyhydroxy compound preferably trioxy propane, particularly glycerine is used for preparing the ester of boric acid and for helping the permeability of the cell membrane.

According to a preferred feature of the process the components are used in the following amounts calculated on the total volume or weight of the composition: 0.01—2.00 % cobalt chloride or the hexahydrate thereof; 0.01—2.00 % ammonium metavanadate; 0.01—2.00 % nickel sulphate or the heptahydrate thereof; 0.001—5.00 % boric acid; 0.01—4.00 % copper sulphate or the pentahydrate thereof; 0.01—10.00 % zinc sulphate or the heptahydrate thereof; 0.01—30.00 % ferrous sulphate or the heptahydrate thereof; 0.01—15.00 % manganese sulphate or the di- or tetrahydrate thereof; 0.01—20.00 % magnesium sulphate or the heptahydrate thereof; 0.01—15.00 % EDTA sodium salt; 0.01—5.00 % succinic anhydride; 0.01—5.00 % succinimide; 0.01—5.00 % succinic monoethyl ester; 0.01—10.00 % glycine (glycocell); 0.01—50.00 % glycerine; 0.01—5.00 % ascorbic acid; 0.01—20.00 % succinic acid.

The activity of the composition was examined in the following animal experiments.

Experiments on sheep:

It has been examined whether any change in the development of lambs consuming the milk of ewes that consumed the composition of the invention during the lactation period occurred. The weight of the

3

sucking lambs was measured prior to the administration of the composition and at the delactation of the lambs. The composition was administered in the drinking-water. For the experiments 40 ewes suffering from "stinking lameness" were used.

| Treatment of ewes | Change in body weight of lambs (kg/animal) | (%) |
|---|---|---|
| Control group (treated with a usual fodder composition) | 4,97 | 100 |
| Group treated with the composition of the invention+the usual composition | 5,84 | 117 |
| Group treated with the composition of the invention+claw treatment (cutting) | 5,87 | 118 |
| $SD_5$ % | 0,87 | 17,5 |

The weight increase of the lambs of the ewes suffering from "stinking lameness" which consumed the composition is 18 % higher than that of the control group, which can be set confirmed.

The weight increase of the lambs of the ewes not suffering from "stinking lameness" was measured as well in two ways. In the first case (A) the joint weight increase of all the lambs and in the second case (B) the weight increase of the lambs having the same weight (5 kg) at the beginning of the experiment were established. The composition was administered to the ewes in their drinking-water. The results of the comparisons A and B are summarized in the following Table.

| Treatment of ewes | Weight increase of the sucking lambs | | | | |
|---|---|---|---|---|---|
| | A | | | B | |
| Control group | 5,40 kg | 100 % | | 5,13 kg | 100 % |
| Group treated with the composition | 6,90 kg | 128 % | | 6,84 kg | 133,3 % |
| $SD_5$ % | 1,33 kg | 24,6 % | | 1,33 kg | 24,6 % |

According to the above results the weight increase of the lambs of ewes not suffering from "stinking lameness" which received the composition of the invention is even more striking. It is by 28 % and 33,3 % higher than that of the control group. The results can be set confirmed.

The effect of the composition of the invention on the weight increase of fatted lambs has been examined as well. Two groups each consisting of 63 similar ram-tegs were used in the experiment. The experiment lasted for 57 days. The results are summarized in the following Table by giving

A: The total weight increase and
B: The weight increase of the lambs having the same weight at the beginning of the experiment.

| | Weight increase | | | |
|---|---|---|---|---|
| | A | | B | |
| Control group | 8,43 kg | 100 % | 8,20 kg | 100 % |
| Group treated with the composition | 9,69 kg | 115 % | 9,80 kg | 120 % |
| $SD_5$ % | | | 0,99 kg | 12,1 % |

The weight increase of the fatted lambs is very significant and it is more striking in the case of the fatted lambs having the same weight at the beginning of the experiment.

According to general experience, the condition of the ewes worsens during the lactation period. This is disadvantageous from the point of view of rutting and re-insemination. In the case of the group suffering from "stinking lameness" the weight of the ewes was measured at the beginning and at the end of the lactation period.

It has been noticed that in case of the control group during the lactation period the weight decrease was on an average of 3.68 kg pro animal whereas in case of the group wherein the ewes consumed the composition of the invention a weight increase of 1.02 kg pro animal was reached. It can be established that by the use of the composition of the invention the condition of the ewes does not worsen, in fact, a weight increase of 1 kg is noticed.

The side effect of the composition of the invention on the prevention of "stinking lameness" has been examined as well on two groups of ewes each consisting of about 40 animals. The treatment lasted for 72 days, 2 ml of the composition were administered daily in the drinking water; the appearance of lameness was examined 8 times. In the following Table the number of the animals which became ill are given.

| | Number of animals | |
|---|---|---|
| Control group | 43 | 107,5 % |
| Group treated with the composition | 8 | 20,0 % |

By the use of the composition the lameness decreased by 87,5 %.

On the basis of the exploratory tests the experiment was carried out for a whole cycle on sheep, turkey-hens, broiler chickens and calves. The keeping conditions were the same in case of the control and treated groups, but the treated animals consumed the composition together with their drinking-water.

At the initial stage of the experiments the following results were obtained.

In the case of turkey-hens wherein the control and treated groups each consisted of 1072 animals the results obtained after 40 days are the following.

| | Control group | Group treated with the composition | Difference |
|---|---|---|---|
| Animals perished and sorted out (in %) | 18 | 11 | 7 |
| Total eggs laid during the 40-days period | 23.430 | 24.934 | 1504 |
| Egg production calculated on one day | 586 | 623 | 37 |
| Hatched turkeys in the % of the incubated eggs | 82,28 | 83,47 | 1,2 |

As it can be seen a significant economical result was achieved by the use of the composition of the invention.

In case of broiler chickens wherein the control and treated groups each consisted of 7000 animals after 26 days the following results were obtained.

| | Control group | Group treated with the composition | Difference |
|---|---|---|---|
| Perished | 209 | 204 | −5 |
| Total fodder consumed in 26 days | 8375 kg | 7525 kg | −850 kg |
| Average weight of 26-days old chickens | 409 g | 502 g | +93 g |

By the use of the composition a significant weight increase and a significant decrease in the amount of fodder needed for the production of 1 kg of live weight were achieved.

In case of calves the control and treated groups each consisted of 22 animals. The most significant result of the experiments was a weight increase of 4 % whilst at the same time the fodder consumption decreased by 20 % and no hoove occured.

| | Control group | Group treated with the composition | Difference |
|---|---|---|---|
| Weight increase g/animal/day | 673 % | 701 % | +4 % |
| Death | 2 | — | −2 |
| Fodder consumption | 100 % | 80 % | −20 % |
| Hoove pro day | 2—3 | 0 | −2—3 |

In case of sheep the control and treated groups each consisted of 500 animals. The results are the following.

| | Control group | Group treated with the composition | Difference |
|---|---|---|---|
| Number of rutting animals | 141 | 167 | +18,4 % |
| Number of transported delactated animals | 310 | 330 | +6,4 % |

As it can be seen the use of the composition exerted an advantageous effect on the rutting of the animals as it significally increased the number of the rutting animals and that of the delactated lambs as well.

The process according to the invention is illustrated by the following Examples.

Example 1

The following components are used, the amounts are calculated on the total volume and weight, resp. of the composition: 0.04 % cobalt chloride or its hexahydrate, 0.046 % ammonium metavanadate, 0.034 % nickel sulphate or its heptahydrate, 0.06 % boric acid, 0.385 % copper sulphate or its pentahydrate, 1.0 % ferrous sulphate or its heptahydrate, 1.76 % zinc sulphate or its heptahydrate, 2.03 % magnesium sulphate or its heptahydrate, 0.852 % manganese sulphate or its dihydrate, 0.672 % EDTA sodium salt, 0.055 % succinic anhydride or its ethyl ester or imide derivative, 0.25 % glycine, 2.00 % glycerine, 0.025 ascorbic acid, 0.2 % succinic acid. For the preparation of the monoethyl succinate 150 ml of ethanol and for that of succinimide 80 ml of 25 % ammonium hydroxide are used. As an inert diluent or carrier preferably water, particularly distilled water is employed.

Example 2

The given amount of cobalt chloride, ammonium metavanadate and succinic anhydride are heated on a water bath in 150 ml of ethanol until all the compounds are dissolved, then the excess of the alcohol is evaporated. The Co-V-ethyl succinate thus obtained is dissolved in water.

Example 3

Succinic anhydride in an amount given in Example 1 is heated and boiled in 40 ml of 25 % ammonium hydroxide solution until the odour of ammonia disappears, while succinimide is formed. To the warm reaction mixture the solution of nickel sulphate heptahydrate (corresponding to the amounts given in Example 1) is added. Water soluble succinimide nickel is obtained.

Example 4

The copper salt and glycine in the amounts given in Example 1 and 652 g of the zinc salt are dissolved in water separately and the warm solutions are combined. Violet-coloured copper and zinc glycinate are obtained.

Example 5

The given amount of manganese sulphate or its hydrate and of EDTA sodium salt are dissolved separately in water under heating. The manganese sulphate solution is acidified with diluted $H_2SO_4$ (to a pH-value of about 6.5) and it is combined with the solution of the EDTA sodium salt. The manganese complex of EDTA is obtained.

Example 6

The given amounts of the ferrous and magnesium salt and of succinic acid as well as 1088 g of the zinc salt are dissolved separately in water and the warm solutions are combined. Ferrous, magnesium and zinc succinates are obtained and a part of the iron, magnesium and zinc remain in the solution in free form.

Example 7

The given amount of boric acid is dissolved in water. 500—500 ml of glycerine is added.

Example 8

A composition (solution) suitable for oral administration is prepared in the following way.

The solutions of the compounds prepared in the preceding Examples are combined in the following succession:

I. Cobalt, vanadium ethyl succinate solution prepared according to Example 2.

II. Nickel succinimide solution prepared according to Example 3.

III. Manganese-EDTA complex solution prepared according to Example 5.

IV. Copper, zinc glycinate complex prepared according to Example 4.

V. Ferrous, magnesium, zinc succinate solution prepared according to Example 6.

VI. The glycerine solution prepared according to Example 7 containing the glycerine ester of boric acid.

To the composition prepared according to Example 8 a reducing agent is added, which is not injurious to the organism of the animals and which does not form a precipitate with the other components. This reducing agent is preferably ascorbic acid which is added in the amount given in Example 1. In case of necessity the pH of the combined solutions is adjusted with a non-toxic, diluted acid, preferably with diluted HCl to a value of 1—3. The composition is administered in a dose corresponding to the species, age and body weight of the animal, preferably in the drinking-water.

According to an alternative method the composition can be obtained by preparing a concentrate from the solution at a temperature of not exceeding 104°C or a solution the density of which is comparable to that of the saturated solution. The concentrate thus obtained is preferably employed by absorbing it on the substances used for feeding. For this purpose preferably grists suitable for feeding are used and a homogenous premix having a moisture content suitable for storing is prepared with these fodder grists.

**Claim**

A process for the preparation of a composition suitable for promoting the utilization of fodder and preventing the impairment of health of alimentary origin of the animals, comprising blending cobalt or vanadium succinate or cobalt or vanadium ethyl succinate, succinimide nickel complex, iron, magnesium or zinc succinate, boric complex of a lower aliphatic polyhydroxy compound, copper or zinc complex of a monoamino-monocarboxylic acid and manganese complex of an aminopolycarboxylic acid, optionally in the presence of a solvent, and processing the mixture into an orally administerable form.

**Patentanspruch**

Verfahren zur Herstellung einer Zusammensetzung zur Erhöhung der Ausnutzung von Futter und zur Verhinderung ernährungsmässig bedingter gesundheitlicher Schäden bei Tieren, gekennzeichnet durch Vermischen von Cobalt- oder Vanadiumsuccinat oder Cobalt- oder Vanadiumethylsuccinat, Succinimid-Nickel-Komplex, Eisen-, Magnesium- oder Zinksuccinat, Borsäurekomplex einer niedrig-aliphatischen Polyhydroxyverbindung, Kupfer- oder Zinkkomplex einer Monoamino-monocarbonsäure und Mangankomplex einer Aminipolycarbonsäure, gegebenenfalls in Anwesenheit eines Lösungsmittels, und Verarbeiten des Gemisches in eine oral zu verabreichende Form.

**Revendication**

Procédé de préparation d'une composition convenant pour l'amélioration de l'utilisation du fourrage et la prévention d'altérations d'origine alimentaire de la santé des animaux, comportant le mélange de succinate de cobalt ou de vanadium ou d'éthyl succinate de cobalt ou de vanadium, d'un complexe du nickel avec le succinimide, de succinate de fer, de magnésium ou de zinc, d'un complexe borique d'un composé polyhydroxylé aliphatique inférieur, d'un complexe du cuivre ou du zinc avec un acide monoamino-monocarboxylique, et d'un complexe du manganèse avec un acide aminopolycarboxylique, éventuellement en présence d'un solvant, et la mise du mélange, par traitement sous une forme qui l'on peut administrer par voie orale.